# FASCICULE DE BREVET EUROPEEN

(11) **EP 0 804 922 B1**
(45) Date de publication et mention de la délivrance du brevet: **07.10.1998**
(21) Numéro de dépôt: 97400536.5
(22) Date de dépôt: 11.03.1997
(51) Int. Cl.: A61K 7/42

(54) **Composition filtrante photostable comprenant un dérivé de dibenzoylméthane et un alkylether de polysaccharide**
Lichtstabile filtrierende Zusammensetzung enthaltend ein Derivat von Dibenzoylmethan und ein Alkyletherpolysaccharid
Photostable screening composition containing a derivative of dibenzoylmethane and an alkyletherpolysaccharide

(30) Priorité: 05.04.1996 FR 9604361
(43) Date de publication de la demande: 05.11.1997
(73) Titulaire: L'OREAL, 75008 Paris (FR)
(72) Inventeur: Hansenne, Isabelle, 75017 Paris (FR); de Chabannes, Karine, 45000 Orleans (FR)
(74) Mandataire: Miszputen, Laurent

(56) Documents cités:
- FR-A- 2 440 933
- RESEARCH DISCLOSURE, no. 37807, Octobre 1995, page 642 XP000549119 MAJEWICZ ET AL.: "OIL-BASED COSMETIC AND THERAPEUTIC COMPOSITIONS CONTAINING ETHYLGUAR"

## Description

La présente invention se rapporte, entre autres objets, à de nouvelles compositions cosmétiques et/ou dermatologiques destinées à la photoprotection de la peau et/ou des cheveux contre le rayonnement ultraviolet (compositions ci-après dénommées plus simplement compositions filtrantes), à leur utilisation dans l'application cosmétique susmentionnée, ainsi qu'à un procédé général de photostabilisation de filtres solaires particuliers, actifs dans l'UV-A, au moyen d'un alkyléther de polysaccharide particulier. Plus précisément encore, elle se rapporte à des compositions filtrantes photostables à l'égard des UV qui comprennent, dans un support cosmétiquement et/ou dermatologiquement acceptable, au moins un composé choisi parmi les dérivés du dibenzoylméthane à titre de filtre solaire organique actif dans l'UV-A, associé à cet alkyléther de polysaccharide particulier à titre d'agent photostabilisant, ainsi qu'au procédé correspondant de stabilisation du dérivé de dibenzoylméthane au moyen de cet alkyléther de polysaccharide.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-(ter.-butyl) 4'-méthoxy dibenzoylméthane, qui présentent en effet un fort pouvoir d'absorption intrinsèque. Ces dérivés du dibenzoylméthane, qui sont maintenant des produits bien connus en soi à titre de filtres actifs dans l'UV-A, sont notamment décrits dans les demandes de brevets français FR-A- 2 326 405 et FR-A- 2 440 933, ainsi que dans la demande de brevet européen EP-A- 0 114 607 ; le 4-(ter.-butyl) 4'-méthoxy dibenzoylméthane est par ailleurs actuellement proposé à la vente sous la dénomination commerciale de "PARSOL 1789" par la Société GIVAUDAN.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV, ce qui est contraignant pour l'utilisateur.

La photostabilisation des dérivés du dibenzoylméthane vis-à-vis du rayonnement UV constitue, à ce jour, un problème qui n'a pas encore été résolu de manière complètement satisfaisante.

Or, la Demanderesse vient maintenant de découvrir, de façon inattendue et surprenante, qu'en associant aux dérivés du dibenzoylméthane mentionnés ci-dessus une quantité efficace d'au moins un alkyléther de polysaccharide particulier, il était possible d'améliorer de manière substantielle et remarquable, la stabilité photochimique (ou photostabilité) de ces mêmes dérivés du dibenzoylméthane.

Cette découverte est à la base de la présente invention.

Ainsi, conformément à un premier objet de la présente invention, il est proposé de nouvelles compositions, plus particulièrement destinées à la protection de la peau et/ou des cheveux contre le rayonnement ultraviolet, du type comprenant, notamment dans un support cosmétiquement acceptable, au moins un dérivé du dibenzoylméthane, caractérisées par le fait qu'elles comprennent en outre au moins un alkyléther de polysaccharide formé de motifs comportant au moins deux cycles osidiques différents, chaque motif comportant au moins un groupe hydroxyle substitué par une chaîne alkyle hydrocarbonée saturée.

Les compositions conformes à l'invention présentent l'avantage d'être particulièrement photostables, même après une exposition prolongée aux rayonnements UV-A et UV-B. Ces rayonnements peuvent être d'origine naturelle (soleil) ou artificielle (lampe UV).

La présente invention a également pour objet un nouveau procédé de stabilisation des dérivés du dibenzoylméthane vis-à-vis du rayonnement UV, consistant à associer auxdits dérivés du dibenzoylméthane une quantité efficace d'au moins un alkyléther de polysaccharide formé de motifs comportant au moins deux cycles osidiques différents, chaque motif comportant au moins un groupe hydroxyle substitué par une chaîne alkyle hydrocarbonée saturée.

La présente invention a également pour objet l'utilisation d'un alkyléther de polysaccharide formé de motifs comportant au moins deux cycles osidiques différents, chaque motif comportant au moins un groupe hydroxyle substitué par une chaîne alkyle hydrocarbonée saturée, pour stabiliser vis-à-vis des rayons UV un dérivé du dibenzoylméthane contenu dans une composition cosmétique filtrante.

Un autre objet encore de la présente invention réside dans un procédé de traitement cosmétique pour la protection de la peau et/ou des cheveux contre le rayonnement ultraviolet, consistant à appliquer sur ces derniers une quantité efficace d'une composition photostable conforme à l'invention.

D'autres caractéristiques, aspects et avantages de la présente invention apparaitront à la lecture de la description détaillée qui va suivre.

Comme indiqué précédemment, les dérivés du dibenzoylméthane destinés à être photostabilisés selon la présente invention sont des produits déjà bien connus en soi et décrits notamment dans les documents FR-A- 2 326 405, FR-A- 2 440 933 et EP-A- 0 114 607 précités.

Selon la présente invention, on peut bien entendu mettre en oeuvre un ou plusieurs dérivés du dibenzoylméthane.

Parmi les dérivés du dibenzoylméthane utilisables selon la présente invention, on peut notamment citer, de manière non limitative :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société GIVAUDAN, ce filtre répondant donc à la formule développée suivante :

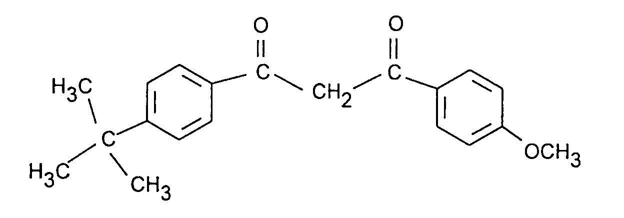

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

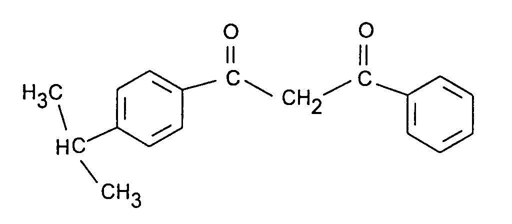

Le ou les dérivés du dibenzoylméthane peuvent être présents dans les compositions conformes à l'invention, ou dans les compositions destinées à être stabilisées conformément au procédé de l'invention, à des teneurs qui sont généralement allant de 0,01 % à 10 % en poids, et de de préférence à des teneurs allant de 0,3 % à 5 % en poids, par rapport au poids total de la composition.

Selon un mode de réalisation particulier de l'invention, l'alkyléther de polysaccharide a un poids moléculaire supérieur à 100 000, et de préférence supérieur à 200 000. Chaque motif peut comporter de un à six et de préférence de deux à quatre groupes hydroxyle substitués par une chaîne alkyle hydrocarbonée saturée.

Par chaîne alkyle hydrocarbonée saturée, on entend une chaîne comportant de 1 à 24, de préférence de 1 à 10 et mieux de 1 à 5 atomes de carbone. En particulier, la chaîne alkyle est choisie parmi les chaînes méthyle, éthyle, n-propyle, isopropyle, n-butyle, isobutyle, tertio-butyle, n-pentyle.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide selon l'invention est un alkyléther d'une gomme et plus particulièrement d'une gomme globalement non ionique, c'est-à-dire pratiquement sans groupe ionique. Comme gommes appropriées, on peut citer par exemple la gomme guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique.

Selon un mode de réalisation préféré de l'invention, l'alkyléther de polysaccharide est un dérivé de gomme guar, et plus particulièrement la guar éthylée ayant un degré de substitution d'environ 2 à 3, en particulier 2,5, telle que décrite dans le document RD 95378007 (octobre 1995).

Par quantité efficace d'alkykéther de polysaccharide selon l'invention, on entend une quantité suffisante pour obtenir une amélioration notable et significative de la photostabilité du ou des dérivés du dibenzoylméthane contenus dans la composition. La quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que celui donné dans les exemples ci-après.

Les compositions selon l'invention peuvent contenir par exemple une quantité d'alkyléther de polysaccharide selon l'invention allant de 0,5 à 20 %, et de préférence de 2 à 10 % du poids total de la composition.

Les compositions cosmétiques filtrantes photostables selon l'invention peuvent bien entendu contenir, outre les dérivés du dibenzoylméthane, un ou plusieurs filtres solaires complémentaires actifs dans l'UVA et/ou l'UVB, hydrophiles ou lipophiles. La présence de filtres complémentaires actifs dans l'UV-B (longueurs d'ondes comprise entre 280nm et 320 nm environ) permet ainsi de disposer de compositions finales capables de filtrer l'ensemble des rayons UV.

Les compositions de l'invention peuvent aussi comprendre des adjuvants cosmétiques et/ou dermatologiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les pigments (minéraux ou organiques), les séquestrants, les polymères, les propulseurs, les agents alcalinisants ou acidifiants, les colorants, ou tout autre ingrédient habituellement utilisé en cosmétique, en particulier pour la fabrication de compositions filtrantes. Bien entendu, tous les ingrédients supplémentaires susceptibles d'être introduits dans les compositions conformes à l'invention doivent être tels qu'ils ne perturbent ou n'altèrent substantiellement pas l'effet de photostabilisation exercé par l'alkyléther de polysaccharide selon l'invention sur les dérivés du dibenzoylméthane.

Les corps gras peuvent être constitués par une huile ou une cire ou leurs mélanges ; ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs ayant moins de 8 atomes de carbone.

Les épaississants autres que l'alkyléther de polysaccharide selon l'invention peuvent être choisis notamment parmi les acides polyacryliques réticulés, les celluloses modifiées ou non, telles que la méthylhydroxyéthylcellulose, l'hydroxypropylméthylcellulose et l'hydroxyéthylcellulose.

Les compositions de l'invention peuvent être préparées selon les techniques bien connues de l'homme de l'art.

Ces compositions peuvent se présenter en particulier sous forme d'une huile, d'une émulsion, simple ou complexe (H/E, E/H, H/E/H ou E/H/E).

De préférence, les compositions de l'invention sont sous la forme d'une huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions cosmétiques photostables de l'invention peuvent être utilisées comme compositions protectrices de l'épiderme humain ou des cheveux contre les rayons ultraviolets, comme compositions filtrantes ou encore comme produits de maquillage.

Lorsque les compositions cosmétiques selon l'invention sont utilisées pour la protection de l'épiderme humain contre les rayons UV ou comme composition filtrante, elles peuvent se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de bâtonnet solide, de stick, de mousse aérosol ou de spray.

Lorsque les compositions cosmétiques selon l'invention sont utilisées pour la protection des cheveux, elles peuvent se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque les compositions sont utilisées comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elles peuvent se présenter sous forme solide ou pâteuse, anhydre ou aqueuse, comme des poudres, des émulsions huile-dans-eau ou eau-dans-huile, des dispersions vésiculaires non ioniques ou encore des suspensions.

Des exemples concrets illustrant l'invention, vont maintenant être donnés.

### EXEMPLE 1 :

On a réalisé les compositions suivantes (les quantités sont exprimées en pourcentage de poids par rapport au poids total de la composition) :

| | |
|---|---|
| - 4-tert-butyl-4'-méthoxydibenzoylméthane (Parsol 1789) | 2 % |
| - benzoate d'alcools (C12/C15) vendu sous la dénomination commerciale « Finsolv TN » par Stéarineries Dubois | 4 % |
| - guar éthylée ayant un degré de substitution d'environ 2,5 | x % |
| - palmitate d'isopropyle | qsp 100 % |

Les différentes compositions, selon la valeur de x, sont rassemblées dans le tableau (I) suivant :

**Tableau (I)**

| **Composition** | **Guar éthylée (x %)** |
|---|---|
| A (comparative) | 0 |
| B (invention) | 4 |
| C (invention) | 7 |

Les compositions ont été réalisées par simple mélange des constituants chauffé à environ 60 °C.

Pour chacune de ces compositions, on a déterminé le pourcentage de 4-tert-butyl-4'-méthoxydibenzoylméthane résiduel après irradiation par l'ensemble du spectre UV (280-400 nm) selon le protocole suivant : pour chaque composition, on a préparé quatre échantillons témoins et quatre échantillons tests. On a déposé sur des plaques de PMMA (polyméthyl méthacrylate) dépolies, préalablement rincées à l'eau puis séchées, 16 mg de composition qu'on a étalée sur une surface de 2 x 4 cm². On a ensuite laissé reposer toutes les plaques une demi-heure à l'obscurité. Puis on a irradié les plaques par l'ensemble du spectre UV (SUNTEST CPS Heraeus) pendant 30 minutes en conservant les plaques témoins à l'obscurité pendant le temps d'irradiation des autres plaques.

On a ensuite dosé les échantillons de la manière suivante : on a procédé à l'extraction des filtres en immergeant chaque plaque dans 50 g d'isopropanol afin de solubiliser les filtres. Les plaques et le solvant contenant les filtres ont ensuite été traités aux ultrasons pendant 5 minutes pour assurer une agitation efficace. La concentration en 4-tert-butyl-4'-méthoxydibenzoylméthane résiduel a été mesurée par spectrophotométrie au maximum d'absorption du 4-tert-butyl-4'-méthoxydibenzoylméthane (355 nm).

Les résultats en pourcentage de la concentration en 4-tert-butyl-4'-méthoxydibenzoylméthane résiduel par rapport à la concentration en 4-tert-butyl-4'-méthoxydibenzoylméthane initiale sont consignés dans le tableau (II) suivant :

**Tableau (II)**

| **Composition** | **Parsol 1789 résiduel** |
|---|---|
| A (comparative) | 67 % |
| B (invention) | 80,5 % |
| C (invention) | 81 % |

Ces résultats montrent clairement que la présence d'un alkyléther de polysaccharide selon l'invention dans une composition contenant du 4-tert-butyl-4'-méthoxydibenzoylméthane (Parsol 1789) augmente de façon significative la stabilité de ce dernier au sein de cette composition.

### EXEMPLE 2 :

On donne ci-dessous un exemple concret d'une huile solaire selon l'invention. Les quantités sont exprimées en poids, par rapport au poids total de la composition.

### Huile solaire :

| | |
|---|---|
| - 4-tert-butyl-4'-méthoxydibenzoylméthane (Parsol 1789) | 1,25 % |
| - α-cyano-β,β' diphénylacrylate de 2-éthylhexyle vendu sous la dénomination commerciale « Uvinul N 539 » par BASF | 3,75 % |
| - guar éthylée ayant un degré de substitution d'environ 2,5 | 4 % |
| - isononanoate d'isononyle de Stéarineries Dubois | qsp 100 % |
| - colorants | qs |
| - parfum | qs |

Cette composition a été réalisée par simple mélange des constituants chauffé à environ 60 °C.

Cette huile solaire est particulièrement photostable et assure une protection dans l'ensemble du rayonnement UV.

## Revendications

1. Composition susceptible de protéger la peau et/ou les cheveux contre le rayonnement ultraviolet, du type comprenant au moins un dérivé du dibenzoylméthane, caractérisée par le fait qu'elle comprend en outre au moins un alkyléther de polysaccharide formé de motifs comportant au moins deux cycles osidiques différents, chaque motif comportant au moins un groupe hydroxyle substitué par une chaîne alkyle hydrocarbonée saturée.

2. Composition selon la revendication 1, caractérisée en ce que chaque motif comporte deux à quatre groupes hydroxyle substitués par une chaîne alkyle hydrocarbonée saturée.

3. Composition la revendication 1 ou 2, caractérisée en ce que la chaîne alkyle hydrocarbonée saturée comporte de 1 à 24 atomes de carbone.

4. Composition selon la revendication 3, caractérisée en ce que la chaîne alkyle hydrocarbonée saturée comporte de 1 à 5 atomes de carbone.

5. Composition la revendication 4, caractérisée en ce que la chaîne alkyle est choisie parmi les chaînes méthyle, éthyle, n-propyle, isopropyle, n-butyle, isobutyle, tertiobutyle.

6. Composition selon l'une quelconque des revendications précédentes, caractérisée en ce que les cycles osidiques sont choisis parmi le mannose, le galactose, le glucose, le furanose, le rhamnose, l'arabinose.

7. Composition selon l'une quelconque des revendications précédentes, caractérisée en ce que l'alkyléther de polysaccharide est un alkyléther d'une gomme choisie parmi la gomme guar, la gomme de caroube, la gomme de karaya, la gomme adragante et leurs mélanges.

8. Composition selon l'une quelconque des revendications précédentes, caractérisée par le fait que l'alkyléther de polysaccharide est de la gomme de guar à chaîne éthyle avec un degré de substitution de 2 à 3.

9. Composition selon l'une quelconque des revendications précédentes, caractérisée en ce que l'alkyléther de polysaccharide a un poids moléculaire supérieur à 200 000.

10. Composition selon l'une quelconque des revendications précédentes, caractérisée par le fait que l'alkyléther de polysaccharide est présent dans la composition à une teneur allant de 0,5 à 20 % en poids, par rapport au poids total de la composition.

11. Composition selon la revendication 10, caractérisée par le fait que cette teneur va de 2 à 10 % en poids, par rapport au poids total de la composition.

12. Composition selon l'une quelconque des revendications précédentes, caractérisée par le fait que le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

13. Composition selon la revendication 12, caractérisée par le fait que le dérivé de dibenzoylméthane est le 4-(ter.-butyl) 4'-méthoxy dibenzoylméthane.

14. Composition selon la revendication 12, caractérisée par le fait que le dérivé de dibenzoylméthane est le 4-isopropyl-dibenzoylméthane.

15. Composition selon l'une quelconque des revendications précédentes, caractérisée par le fait que le dérivé de dibenzoylméthane est présent dans la composition à une teneur allant de 0,01 % à 10 % en poids, par rapport au poids total de la composition.

16. Composition selon la revendication 15, caractérisée par le fait que cette teneur va de 0,3 % à 5 % en poids, par rapport au poids total de la composition.

17. Composition selon l'une quelconque des revendications précédentes, caractérisée par le fait qu'elle comprend en outre un support cosmétiquement et/ou dermatologiquement acceptable.

18. Composition selon l'une quelconque des revendications précédentes, caractérisée par le fait qu'elle est sous la forme d'une huile.

19. Composition selon l'une quelconque des revendications précédentes, caractérisée par le fait qu'elle est photostable.

20. Procédé de stabilisation des dérivés du dibenzoylméthane vis-à-vis du rayonnement UV, caractérisé par le fait qu'il consiste à associer auxdits dérivés du dibenzoylméthane une quantité efficace d'au moins un alkyléther de polysaccharide formé de motifs comportant au moins deux cycles osidiques différents, chaque motif comportant au moins un groupe hydroxyle substitué par une chaîne alkyle hydrocarbonée saturée.

21. Procédé selon la revendication 20, caractérisé en ce que chaque motif comporte deux à quatre groupes hydroxyle substitués par une chaîne alkyle hydrocarbonée saturée.

22. Procédé selon la revendication 20 ou 21, caractérisé en ce que la chaîne alkyle hydrocarbonée saturée comporte de 1 à 24 atomes de carbone.

23. Procédé selon la revendication 22, caractérisé en ce que la chaîne alkyle hydrocarbonée saturée comporte de 1 à 5 atomes de carbone.

24. Procédé la revendication 23, caractérisé en ce que la chaîne alkyle est choisie parmi les chaînes méthyle, éthyle, n-propyle, isopropyle, n-butyle, isobutyle, tertiobutyle.

25. Procédé selon l'une quelconque des revendications 20 à 24, caractérisé en ce que les cycles osidiques sont choisis parmi le mannose, le galactose, le glucose, le furanose, le rhamnose, l'arabinose.

26. Procédé selon l'une quelconque des revendications 20 à 25, caractérisé en ce que l'alkyléther de polysaccharide est un alkyléther d'une gomme choisie parmi la gomme guar, la gomme de caroube, la gomme de karaya, la gomme adragante et leurs mélanges.

27. Procédé selon l'une quelconque des revendications 20 à 26, caractérisé en ce que l'alkyléther de polysaccharide est de la gomme de guar à chaîne éthyle avec un degré de substitution de 2 à 3.

28. Procédé selon l'une quelconque des revendications 20 à 27, caractérisé en ce que l'alkyléther de polysaccharide a un poids moléculaire supérieur à 200 000.

29. Procédé selon l'une quelconque des revendications 20 à 28, caractérisé par le fait que le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-d iméthyl-4-tert.-butyl-4'-méthoxydibenzoylméthane.

30. Procédé selon la revendication 29, caractérisé par le fait que le dérivé de dibenzoylméthane est le 4-(ter.-butyl) 4'-méthoxy dibenzoylméthane.

31. Procédé selon la revendication 29, caractérisé par le fait que le dérivé de dibenzoylméthane est le 4-isopropyl-dibenzoylméthane.

32. Utilisation d'un alkyléther de polysaccharide formé de motifs comportant au moins deux cycles osidiques différents, chaque motif comportant au moins un groupe hydroxyle substitué par une chaîne alkyle hydrocarbonée saturée, pour stabiliser vis-à-vis des rayons UV un dérivé du dibenzoylméthane contenu dans une composition filtrante.

33. Utilisation selon la revendication 32, caractérisée en ce que chaque motif comporte deux à quatre groupes hydroxyle substitués par une chaîne alkyle hydrocarbonée saturée.

34. Utilisation selon la revendication 32 ou 33, caractérisée en ce que la chaîne alkyle hydrocarbonée saturée comporte de 1 à 24 atomes de carbone.

35. Utilisation selon la revendication 34, caractérisée en ce que la chaîne alkyle hydrocarbonée saturée comporte de 1 à 5 atomes de carbone.

36. Utilisation la revendication 35, caractérisée en ce que la chaîne alkyle est choisie parmi les chaînes méthyle, éthyle, n-propyle, isopropyle, n-butyle, isobutyle, tertiobutyle.

37. Utilisation selon l'une quelconque des revendications 32 à 36, caractérisée en ce que les cycles osidiques sont choisis parmi le mannose, le galactose, le glucose, le furanose, le rhamnose, l'arabinose.

38. Utilisation selon l'une quelconque des revendications 32 à 37, caractérisée en ce que l'alkyléther de polysaccharide est un alkyléther d'une gomme choisie parmi la gomme guar, la gomme de caroube, la gomme de karaya, la gomme adragante et leurs mélanges.

39. Utilisation selon l'une quelconque des revendications 32 à 38, caractérisée en ce que l'alkyléther de polysaccharide est de la gomme de guar à chaîne éthyle avec un degré de substitution de 2 à 3.

40. Utilisation selon l'une quelconque des revendications 32 à 39, caractérisée en ce que l'alkyléther de polysaccharide a un poids moléculaire supérieur à 200 000.

41. Utilisation selon l'une quelconque des revendications 32 à 40, caractérisée par le fait que le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

42. Utilisation selon la revendication 41 caractérisée par le fait que le dérivé de dibenzoylméthane est le 4-(ter.-butyl) 4'-méthoxy dibenzoylméthane.

43. Utilisation selon la revendication 41, caractérisée par le fait que le dérivé de dibenzoylméthane est le 4-isopropyl-dibenzoylméthane.

44. Procédé de traitement cosmétique pour la protection de la peau et/ou des cheveux contre le rayonnement ultraviolet, consistant à appliquer sur la peau et/ou les cheveux une quantité efficace d'une composition telle que définie à l'une quelconque des revendications 1 à 19.

## Claims

1. Composition capable of protecting the skin and/or the hair against ultraviolet radiation, of the type comprising at least one dibenzoylmethane derivative, characterized in that it also comprises at least one polysaccharide alkyl ether formed of units containing at least two different monosaccharide rings, each unit containing at least one hydroxyl group substituted with a saturated hydrocarbon alkyl chain.

2. Composition according to Claim 1, characterized in that each unit contains two to four hydroxyl groups substituted with a saturated hydrocarbon alkyl chain.

3. Composition according to Claim 1 or 2, characterized in that the saturated hydrocarbon alkyl chain contains from 1 to 24 carbon atoms.

4. Composition according to Claim 3, characterized in that the saturated hydrocarbon alkyl chain contains from 1 to 5 carbon atoms.

5. Composition according to Claim 4, characterized in that the alkyl chain is chosen from methyl, ethyl, n-propyl, isopropyl, n-butyl, isobutyl and tert-butyl chains.

6. Composition according to any one of the preceding claims, characterized in that the monosaccharide rings are chosen from mannose, galactose, glucose, furanose, rhamnose and arabinose.

7. Composition according to any one of the preceding claims, characterized in that the polysaccharide alkyl ether is an alkyl ether of a gum chosen from guar gum, carob gum, karaya gum, gum tragacanth and mixtures thereof.

8. Composition according to any one of the preceding claims, characterized in that the polysaccharide alkyl ether is a guar gum containing an ethyl chain, with a degree of substitution of 2 to 3.

9. Composition according to any one of the preceding claims, characterized in that the polysaccharide alkyl ether has a molecular weight greater than 200,000.

10. Composition according to any one of the preceding claims, characterized in that the polysaccharide alkyl ether is present in the composition at a content ranging from 0.5 to 20% by weight relative to the total weight of the composition.

11. Composition according to Claim 10, characterized in that this content ranges from 2 to 10% by weight relative to the total weight of the composition.

12. Composition according to any one of the preceding claims, characterized in that the dibenzoylmethane derivative is chosen from:
- 2-methyldibenzoylmethane
- 4-methyldibenzoylmethane
- 4-isopropyldibenzoylmethane
- 4-tert-butyldibenzoylmethane
- 2,4-dimethyldibenzoylmethane
- 2,5-dimethyldibenzoylmethane
- 4,4'-diisopropyldibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

13. Composition according to Claim 12, characterized in that the dibenzoylmethane derivative is 4-(tert-butyl)-4'-methoxydibenzoylmethane.

14. Composition according to Claim 12, characterized in that the dibenzoylmethane derivative is 4-isopropyldibenzoylmethane.

15. Composition according to any one of the preceding claims, characterized in that the dibenzoylmethane derivative is present in the composition at a content ranging from 0.01% to 10% by weight relative to the total weight of the composition.

16. Composition according to Claim 15, characterized in that this content ranges from 0.3% to 5% by weight relative to the total weight of the composition.

17. Composition according to any one of the preceding claims, characterized in that it also comprises a cosmetically and/or dermatologically acceptable vehicle.

18. Composition according to any one of the preceding claims, characterized in that it is in the form of an oil.

19. Composition according to any one of the preceding claims, characterized in that it is photostable.

20. Process for the stabilization of dibenzoylmethane derivatives with respect to UV radiation, characterized in that it consists in combining with the said dibenzoylmethane derivatives an effective amount of at least one polysaccharide alkyl ether formed of units containing at least two different monosaccharide rings, each unit containing at least one hydroxyl group substituted with a saturated hydrocarbon alkyl chain.

21. Process according to Claim 20, characterized in that each unit contains two to four hydroxyl groups substituted with a saturated hydrocarbon alkyl chain.

22. Process according to Claim 20 or 21, characterized in that the saturated hydrocarbon alkyl chain contains from 1 to 24 carbon atoms.

23. Process according to Claim 22, characterized in that the saturated hydrocarbon alkyl chain contains from 1 to 5 carbon atoms.

24. Process according to Claim 23, characterized in that the alkyl chain is chosen from methyl, ethyl, n-propyl, isopropyl, n-butyl, isobutyl and tert-butyl chains.

25. Process according to any one of Claims 20 to 24, characterized in that the monosaccharide rings are chosen from mannose, galactose, glucose, furanose, rhamnose and arabinose.

26. Process according to any one of Claims 20 to 25, characterized in that the polysaccharide alkyl ether is an alkyl ether of a gum chosen from guar gum, carob gum, karaya gum, gum tragacanth and mixtures thereof.

27. Process according to any one of Claims 20 to 26, characterized in that the polysaccharide alkyl ether is a guar gum containing an ethyl chain, with a degree of substitution of 2 to 3.

28. Process according to any one of Claims 20 to 27, characterized in that the polysaccharide alkyl ether has a molecular weight greater than 200,000.

29. Process according to any one of Claims 20 to 28, characterized in that the dibenzoylmethane derivative is chosen from:
- 2-methyldibenzoylmethane
- 4-methyldibenzoylmethane
- 4-isopropyldibenzoylmethane
- 4-tert-butyldibenzoylmethane
- 2,4-dimethyldibenzoylmethane
- 2,5-dimethyldibenzoylmethane
- 4,4'-diisopropyldibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

30. Process according to Claim 29, characterized in that the dibenzoylmethane derivative is 4-(tert-butyl)-4'-methoxydibenzoylmethane.

31. Process according to Claim 29, characterized in that the dibenzoylmethane derivative is 4-isopropyldibenzoylmethane.

32. Use of a polysaccharide alkyl ether formed of units containing at least two different monosaccharide rings, each unit containing at least one hydroxyl group substituted with a saturated hydrocarbon alkyl chain, in order to stabilize a dibenzoylmethane derivative with respect to UV radiation which is comprised in a screening composition.

33. Use according to claim 32, characterized in that each unit contains two or four hydroxyl groups substituted with a saturated hydrocarbon alkyl chain

34. Use according to claim 32 or 33, characterized in that the saturated hydrocarbon alkyl chain contains from 1 to 24 carbon atoms.

35. Use according to claim 34, characterized in that the saturated hydro-carbon alkyl chain contains from 1 to 5 carbon atoms.

36. Use according to claim 35, characterized in that, the alkyl chain is chosen from methyl, ethyl, n-propyl, isopropyl, n-butyl, isobutyl and tertiobutyl chains.

37. Use according to any one of claims 32 to 36, characterized in that the monosaccharide rings are chosen from mannose, galactose, glucose, furanose, rhamnose and arabinose.

38. Use according to any one of claims 32 to 37, characterized in that the the polysaccharide alkyl ether is an alkyl ether of a gum chosen from guar gum, caroube gum, karaya gum, gum tragacanth and mixtures therof.

39. Use according to any one of claims 32 to 38, characterized in that the the polysaccharide alkyl ether is a guar gum containing an ethyl chain with a degree of substitution of 2 to 3.

40. Use according to any one of claims 32 to 39, characterized in that the polysaccharide alkyl ether has a molecular weight greater than 200,000.

41. Use according to any one of claims 32 to 40, characterized in that the dibenzoylméthane dérivative is chosen from ;
- 2-méthyldibenzoylméthane
- 4-méthyldibenzoylméthane
- 4-isopropyldibenzoylméthane
- 4-tert-butyldibenzoylméthane
- 2,4-diméthyldibenzoylméthane
- 2,5-diméthyldibenzoylméthane
- 4,4'-diisopropyldibenzoylméthane.
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

42. Use according to Claim 41, characterized in that the dibenzoylmethane derivative is 4-(tert-butyl)-4'-methoxydibenzoylmethane.

43. Use according to Claim 41, characterized in that the dibenzoylmethane derivative is 4-isopropyldibenzoylmethane.

44. Cosmetic treatment process for protecting the skin and/or the hair against ultraviolet radiation, this process consisting in applying an effective amount of a composition as defined in any one of Claims 1 to 19 to the skin and/or the hair.

## Patentansprüche

1. Zusammensetzungen, die befähigt sind, die Haut und/oder die Haare gegen UV-Strahlung zu schützen, und die mindestens ein Dibenzoylmethanderivat enthalten,
dadurch gekennzeichnet, daß
sie ferner mindestens einen Polysaccharidalkylether enthalten, der aus Einheiten gebildet wird, die mindestens zwei unterschiedliche Zuckerringe enthalten, wobei jede Einheit mindestens eine Hydroxygruppe aufweist, die mit einer Alkylgruppe substituiert ist.

2. Zusammensetzungen nach Anspruch 1, dadurch gekennzeichnet, daß jede Einheit zwei bis vier Hydroxygruppen aufweist, die mit einer Alkylgruppe substituiert sind.

3. Zusammensetzung nach Anspruch 1 oder 2, dadurch gekennzeichnet, daß die Alkylgruppe 1 bis 24 Kohlenstoffatome aufweist.

4. Zusammensetzung nach Anspruch 3, dadurch gekennzeichnet, daß die Alkylgruppe 1 bis 5 Kohlenstoffatome aufweist.

5. Zusammensetzung nach Anspruch 4, dadurch gekennzeichnet, daß die Alkylgruppe unter Methyl, Ethyl, n-Propyl, Isopropyl, n-Butyl, Isobutyl und *tert*.-Butyl ausgewählt ist.

6. Zusammensetzung nach einem der hervorgehenden Ansprüche, dadurch gekennzeichnet, daß die Zuckerringe unter Mannose, Galactose, Glucose, Furanose, Rhamnose und Arabinose ausgewählt sind.

7. Zusammensetzung nach einem der vorhergehenden Ansprüche, dadurch gekennzeichnet, daß der Polysaccharidalkylether ein Alkylether eines Gummis ist, das unter Guargummi, Carubin, Karaya-Gummi, Tragant und deren Gemischen ausgewählt ist.

8. Zusammensetzungen nach einem der vorhergehenden Ansprüche, dadurch gekennzeichnet, daß der Polysaccharidalkylether ein Guargummi mit Ethylgruppe ist, das einen Substitutionsgrad von 2 bis 3 aufweist.

9. Zusammensetzungen nach einem der vorhergehenden Ansprüche, dadurch gekennzeichnet, daß der Polysaccharidalkylether ein Molekülgewicht über 200 000 aufweist.

10. Zusammensetzungen nach einem der vorhergehenden Ansprüche, dadurch gekennzeichnet, daß der Polysaccharidalkylether in der Zusammensetzung in einem Mengenanteil von 0,5 bis 20 Gew.-%, bezogen auf das Gesamtgewicht der Zusammensetzung, vorliegt.

11. Zusammensetzung nach Anspruch 10, dadurch gekennzeichnet, daß der Mengenanteil im Bereich von 2 bis 10 Gew.-%, bezogen auf das Gesamtgewicht der Zusammensetzung, liegt.

12. Zusammensetzung nach einem der vorhergehenden Ansprüche, dadurch gekennzeichnet, daß das Dibenzoylmethanderivat ausgewählt ist unter
- 2-Methyldibenzoylmethan,
- 4-Methyldibenzoylmethan,
- 4-Isopropyldibenzoylmethan,
- 4-(*tert*.-Butyl)-dibenzoylmethan,
- 2,4-Dimethyldibenzoylmethan,
- 2,5-Dimethyldibenzoylmethan,
- 4,4'-Diisopropyldibenzoylmethan,
- 4-(*tert*.-Butyl)-4'-methoxydibenzoylmethan,
- 2-Methyl-5-isopropyl-4'-methoxydibenzoylmethan,
- 2-Methyl-5-(*tert*.-butyl)-4'-methoxydibenzoylmethan,
- 2,4-Dimethyl-4'-methoxydibenzoylmethan,
- 2,6-Dimethyl-4-(*tert*.-butyl)-4'-methoxydibenzoylmethan.

13. Zusammensetzung nach Anspruch 12, dadurch gekennzeichnet, daß das Dibenzoylmethanderivat das 4-(*tert*.-Butyl)-4'-methoxydibenzoylmethan ist.

14. Zusammensetzung nach Anspruch 12, dadurch gekennzeichnet, daß das Dibenzoylmethanderivat das 4-Isopropyldibenzoylmethan ist.

15. Zusammensetzung nach einem der vorhergehenden Ansprüche, dadurch gekennzeichnet, daß das Dibenzoylmethanderivat in der Zusammensetzung in einem Mengenanteil von 0,01 bis 10 Gew.-%, bezogen auf das Gesamtgewicht der Zusammensetzung, vorliegt.

16. Zusammensetzung nach Anspruch 15, dadurch gekennzeichnet, daß der Mengenanteil im Bereich von 0,3 bis 5 Gew.-%, bezogen auf das Gesamtgewicht der Zusammensetzung, liegt.

17. Zusammensetzung nach einem der vorhergehenden Ansprüche, dadurch gekennzeichnet, daß sie ferner einen kosmetisch und/oder dermatologisch akzeptablen Träger aufweist.

18. Zusammensetzung nach einem der vorhergehenden Ansprüche, dadurch gekennzeichnet, daß sie in Form eines Öls vorliegt.

19. Zusammensetzung nach einem der vorhergehenden Ansprüche, dadurch gekennzeichnet, daß sie photostabil sind.

20. Verfahren zur Stabilisierung von Dibenzoylmethanderivaten gegenüber UV-Strahlung, dadurch gekennzeichnet, daß es darin besteht, die Dibenzoylmethanderivate mit einer wirksamen Menge mindestens eines Polysaccharidalkylethers zu kombinieren, der aus Einheiten gebildet wird, die mindestens zwei unterschiedliche Zuckerringe enthalten, wobei jede Einheit mindestens eine Hydroxygruppe aufweist, die mit einer Alkylgruppe substituiert ist.

21. Verfahren nach Anspruch 20, dadurch gekennzeichnet, daß jede Einheit zwei bis vier Hydroxygruppen aufweist, die mit einer Alkylgruppe substituiert sind.

22. Verfahren nach Anspruch 20 oder 21, dadurch gekennzeichnet, daß die Alkylgruppe 1 bis 24 Kohlenstoffatome aufweist.

23. Verfahren nach Anspruch 22, dadurch gekennzeichnet, daß die Alkylgruppe 1 bis 5 Kohlenstoffatome aufweist.

24. Verfahren nach Anspruch 23, dadurch gekennzeichnet, daß die Alkylgruppe unter Methyl, Ethyl, n-Propyl, Isopropyl, n-Butyl, Isobutyl und *tert*.-Butyl ausgewählt ist.

25. Verfahren nach einem der Ansprüche 20 bis 24, dadurch gekennzeichnet, daß die Zuckerringe unter Mannose, Galactose, Glucose, Furanose, Rhamnose und Arabinose ausgewählt sind.

26. Verfahren nach einem der Ansprüche 20 bis 25, dadurch gekennzeichnet, daß der Polysaccharidalkylether ein Alkylether eines Gummis ist, das unter Guargummi, Carubin, Karayagummi, Tragant und deren Gemischen ausgewählt ist.

27. Verfahren nach einem der Ansprüche 20 bis 26, dadurch gekennzeichnet, daß der Polysaccharidalkylether ein Guargummi mit Ethylgruppe ist, das einen Substitutionsgrad von 2 bis 3 aufweist.

28. Verfahren nach einem der Ansprüche 20 bis 27, dadurch gekennzeichnet, daß der Polysaccharidalkylether ein Molekülgewicht über 200 000 aufweist.

29. Verfahren nach einem der Ansprüche 20 bis 28, dadurch gekennzeichnet, daß das Dibenzoylmethanderivat ausgewählt ist unter:
- 2-Methyldibenzoylmethan,
- 4-Methyldibenzoylmethan,
- 4-Isopropyldibenzoylmethan,
- 4-(*tert*.-Butyl)-dibenzoylmethan,
- 2,4-Dimethyldibenzoylmethan,
- 2,5-Dimethyldibenzoylmethan,
- 4,4'-Diisopropyldibenzoylmethan,
- 4-(*tert*.-Butyl)-4'-methoxydibenzoylmethan,
- 2-Methyl-5-isopropyl-4'-methoxydibenzoylmethan,
- 2-Methyl-5-(*tert*.-butyl)-4'-methoxydibenzoylmethan,
- 2,4-Dimethyl-4'-methoxydibenzoylmethan,
- 2,6-Dimethyl-4-(*tert*.-butyl)-4'-methoxydibenzoylmethan.

30. Verfahren nach Anspruch 29, dadurch gekennzeichnet, daß das Dibenzoylmethanderivat das 4-(*tert*.-Butyl)-4'-methoxydibenzoylmethan ist.

31. Verfahren nach Anspruch 29, dadurch gekennzeichnet, daß das Dibenzoylmethanderivat das 4-Isopropyldibenzoylmethan ist.

32. Verwendung eines Polysaccharidalkylethers, der aus Einheiten gebildet wird, die mindestens zwei unterschiedliche Zuckerringe enthalten, wobei jede Einheit mindestens eine Hydroxygruppe aufweist, die mit einer Alkylgruppe substituiert ist, zur Stabilisierung eines in einer Filterzusammensetzung enthaltenen Dibenzoylmethanderivats gegenüber UV-Strahlung.

33. Verwendung nach Anspruch 32, dadurch gekennzeichnet, daß jede Einheit zwei bis vier Hydroxygruppen aufweist, die mit einer Alkylgruppe substituiert sind.

34. Verwendung nach Anspruch 32 oder 33, dadurch gekennzeichnet, daß die Alkylgruppe 1 bis 24 Kohlenstoffatome aufweist.

35. Verwendung nach Anspruch 34, dadurch gekennzeichnet, daß die Alkylgruppe 1 bis 5 Kohlenstoffatome aufweist.

36. Verwendung nach Anspruch 35, dadurch gekennzeichnet, daß die Alkylgruppe unter Methyl, Ethyl, n-Propyl, Isopropyl, n-Butyl, Isobutyl und *tert*.-Butyl ausgewählt ist.

37. Verwendung nach einem der Ansprüche 32 bis 36, dadurch gekennzeichnet, daß die Zuckerringe unter Mannose, Galactose, Glucose, Furanose, Rhamnose und Arabinose ausgewählt sind.

38. Verwendung nach einem der Ansprüche 32 bis 37, dadurch gekennzeichnet, daß der Polysaccharidalkylether ein Alkylether eines Gummis ist, das unter Guargummi, Carubin, Karayagummi, Tragant und deren Gemischen ausgewählt ist.

39. Verwendung nach einem der Ansprüche 32 bis 38, dadurch gekennzeichnet, daß der Polysaccharidalkylether ein Guargummi mit Ethylgruppe ist, das einen Substitutionsgrad von 2 bis 3 aufweist.

40. Verwendung nach einem der Ansprüche 32 bis 39, dadurch gekennzeichnet, daß der Polysaccharidalkylether ein Molekülgewicht über 200 000 aufweist.

41. Verwendung nach einem der Ansprüche 32 bis 40, dadurch gekennzeichnet, daß das Dibenzoylmethanderivat ausgewählt ist unter:
- 2-Methyldibenzoylmethan,
- 4-Methyldibenzoylmethan,
- 4-Isopropyldibenzoylmethan,
- 4-(*tert*.-Butyl)-dibenzoylmethan,
- 2,4-Dimethyldibenzoylmethan,
- 2,5-Dimethyldibenzoylmethan,
- 4,4'-Diisopropyldibenzoylmethan,
- 4-(*tert*.-Butyl)-4'-methoxydibenzoylmethan,
- 2-Methyl-5-isopropyl-4'-methoxydibenzoylmethan,
- 2-Methyl-5-(*tert*.-butyl)-4'-methoxydibenzoylmethan,
- 2,4-Dimethyl-4'-methoxydibenzoylmethan,
- 2,6-Dimethyl-4-(*tert*.-butyl)-4'-methoxydibenzoylmethan.

42. Verwendung nach Anspruch 41, dadurch gekennzeichnet, daR das Dibenzoylmethanderivat das 4-(*tert*.-Butyl)-4'-methoxydibenzoylmethan ist.

43. Verwendung nach Anspruch 41, dadurch gekennzeichnet, daß das Dibenzoylmethanderivat das 4-Isopropyldibenzoylmethan ist.

44. Verfahren zur kosmetischen Behandlung zum Schutz der Haut und/oder der Haare gegen UV-Strahlung, das darin besteht, auf die Haut und/oder die Haare eine wirksame Menge einer Zusammensetzung nach einem der Ansprüche 1 bis 19 aufzutragen.
